# EUROPEAN PATENT APPLICATION

(11) **EP 2 508 139 A1**
(43) Date of publication of application: **10.10.2012**
(21) Application number: 10834523.2
(22) Date of filing: 26.11.2010
(51) Int. Cl.: A61B 17/12

(54) **SURGICAL CLIP**

(30) Priority: 04.12.2009 JP 2009008627 U
(71) Applicant: Nakamura, Shoichi, Higashichikuma-gun, Nagano 399-7502 (JP); ACP Japan Co. Ltd., Tokyo 113-0033 (JP)
(72) Inventor: NAKAMURA, Shoishi, Nagano, 399-7502 (JP)
(74) Representative: Gassner, Wolfgang
(86) International application number: PCT/JP2010/071078
(87) International publication number: WO 2011/068073

(57) **Abstract**

The present invention is to offer such a surgical clip enabling to be attached to and detached from safely and easily even without using a forceps. The surgical clip according to this invention has a clipping portion 20 having a pair of feet 11A, 11B for holding an object, a crossed portion 30 of the feet 11A, 11B crossing each other, and a gripper 40 coupling the crossing feet 11A, 11B in loop and releasing the clipping portion by gripping on its outside. Further, this surgical clip 10 has a balancer 50 connecting to the gripper 40.

## Description

### TECHNICAL FIELD

The present invention relates to a surgical clip used for chiefly clipping a blood vessel to control blood flow, in particular, such a surgical clip easily opening and closing without a forceps.

### BACKGROUND OF THE INVENTION

The surgical clip has been conventionally used to blood vessel anastomoses in a micro surgery. The surgical clip is also called as a bulldog forceps, and it serves clipping blood vessels of an artery or a vein to press them for controlling bleeding or holding hemostatis. Such a surgical clip is also used to keep clipping a suture thread at its end point not to miss it during an operation.

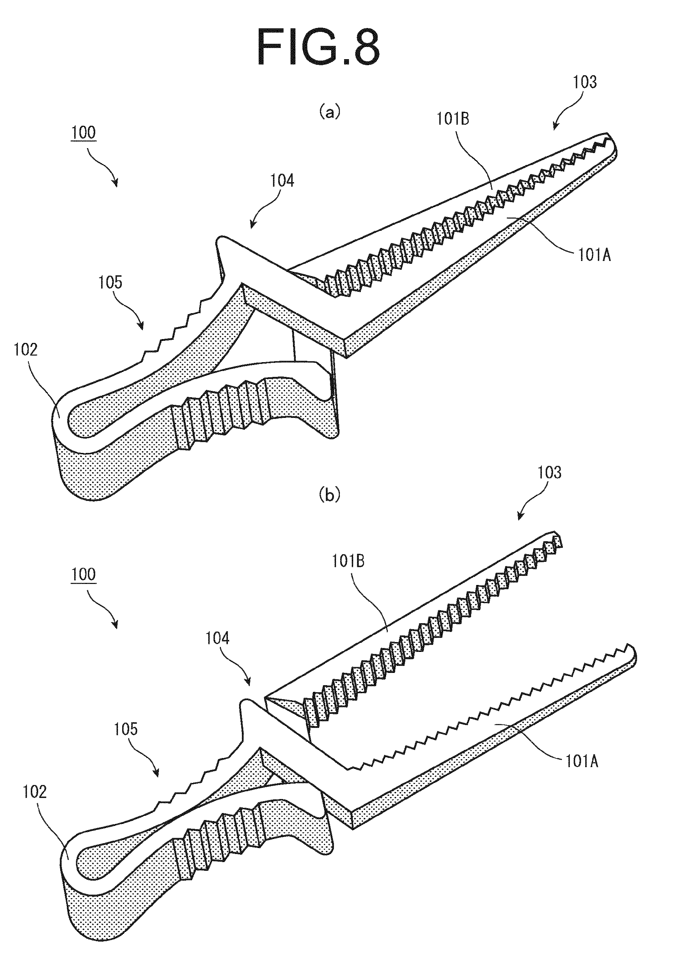

FIG. 8 shows one example of the foregoing surgical clip. FIG. 8(a) illustrates that the surgical clip is closed, and FIG. 8(b) is its opening. As shown in them, the foregoing surgical clip 100 is composed of a pair of feet 101A, 101B curving and continuing at a curve 102. The feet 101A, 101B form a clipping portion 103 clipping an object, a crossed portion 104 giving clipping force to the clipping portion 103 owing to elasticity of the member materials by crossing, and a gripper 105 which is almost loop-shaped and releases the clipping portion 103 by holding it at an outside to give force.

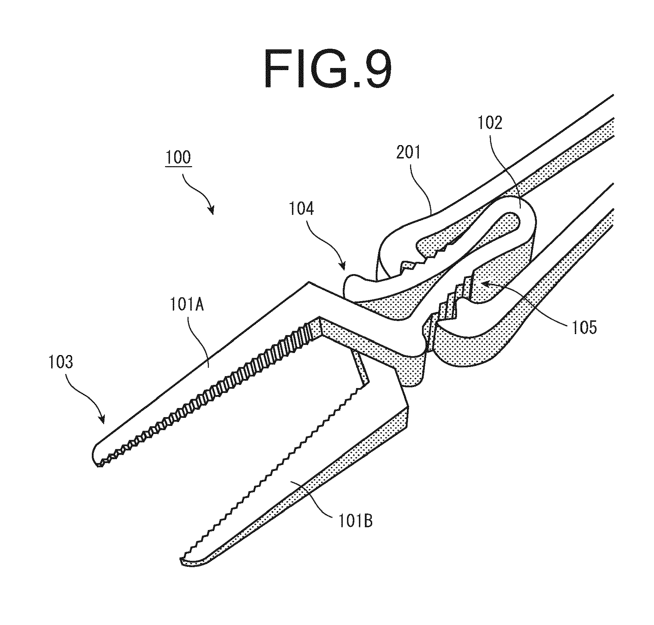

In general, for clipping the object by the surgical clip 100, as seeing in FIG. 9, the forceps 201 holds the gripper 105 on the outside and presses it toward the inside to open the feet 101A, 101B of the clipping part 103 to work.

### SUMMARY OF THE INVENTION

### PROBLEMS THAT THE INVENTION IS TO SOLVE

As mentioned above, when the foregoing surgical clip 100 clips the object and removes it from the clipping state, it each time and certainly necessitates the forceps 201. Therefore, it has had an intricate problem in handling.

Actually, it is possible to open the surgical clip 100 by hand, not using the forceps 201. However, the surgical clip 100 is usually small in size as from several millimeters to several ten millimeters, and in case the gripper 105 given by force when opening a clipping part 103, has an inclined structure, it is unstable when held by a hand, and it easily slips when giving force. Especially, in the case of the surgical clip 100 having a shape as shown in FIG. 8, since a gripper 105 has a wide interior (a side of the clipping part 103) and a narrow front (the side of a curve 102), and if a hand slips, this surgical clip 100 easily splits toward the clipping side of the object. For this reason, the forceps 201 has been indispensable. This fact is the same also in the case of clipping at an end of the suture thread.

Therefore, in view of the above mentioned existing problems, the present invention is to offer such a surgical clip enabling to be attached to and detached from safely and easily even without using the forceps.

### MEANS FOR SOLVING THE PROBLEMS

In consideration of settling the above mentioned problems, the surgical clip according to this invention is characterized by providing a clipping portion having a pair of feet for holding an object therebetween, a crossed portion of the feet crossing each other, and a gripper coupling the crossing feet in loop-shape and releasing the clipping portion by gripping on its outside, wherein the crossing feet are connected in loop-shape and a bridged portion connected in loop-shape is the gripper. Further, this surgical clip has a balancer connecting to the gripper.

Herein, the present surgical clip has sufficiently a weight in the balancer. In such a case, preferably, the balancer is made of a ring shaped member, and the weight has a ball shape to be fitted in the ring shaped member. The surgical clip is formed in combination of plural pieces of plastic materials including a silicone resin.

### EFFECTS OF THE INVENTION

According to the invention, it is possible to realize such a surgical clip of a simple structure, enabling to safely and easily attach to and detach from even without using the forceps, and being smooth finger work and less slippery than the conventional surgical clip.

### BRIEF DESCRIPTION OF THE DRAWINGS

[FIG. 1] A perspective view illustrating a closing condition of the surgical clip according to one embodiment of the invention;
[FIG. 2] A perspective view illustrating an opening condition of the surgical clip of FIG. 1;

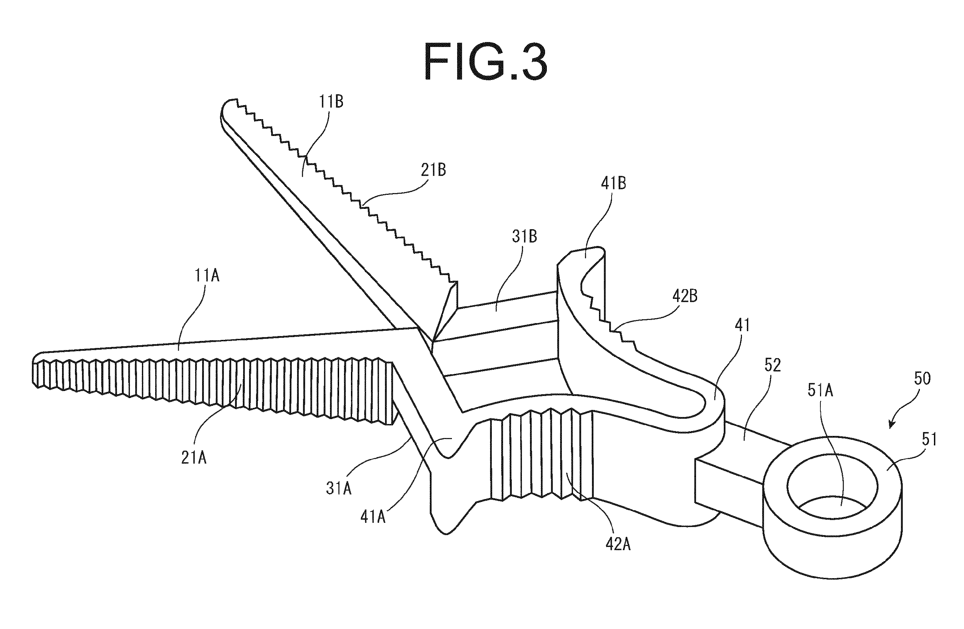

[FIG. 3] A perspective view of a condition before crossing the surgical clip of FIG. 1 at a crossing portion;

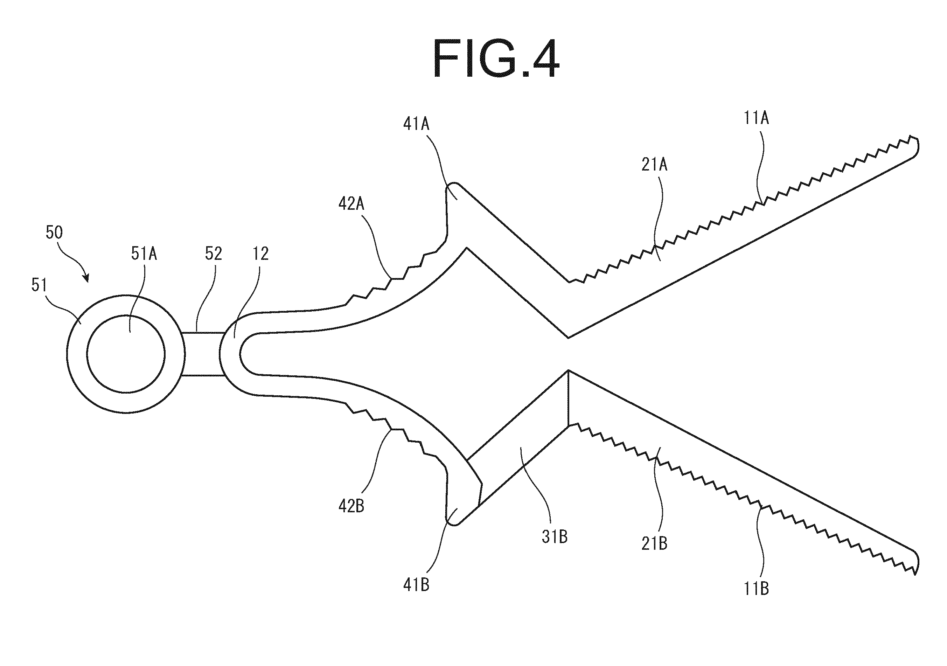

[FIG. 4] A top surface of the surgical clip of FIG. 3;
[FIG. 5] A typical view of gripping the surgical clip of FIG. 1 by operator's fingers;
[FIG. 6] A perspective view of closing the surgical clip according to the other embodiment of the invention;
[FIG. 7] A typical view of serving the surgical clip of FIG. 6 to clip a suture thread at its end part;
[FIG. 8] A schematic view showing one example of a conventional surgical clip; and
[FIG. 9] A schematic view showing the example of using the conventional surgical clip shown in FIG. 8.

### EMBODIMENTS FOR PRACTISING THE INVENTION

In the following, with respect to the embodiment of this invention, detailed explanation will be made, referring to the attached drawings.

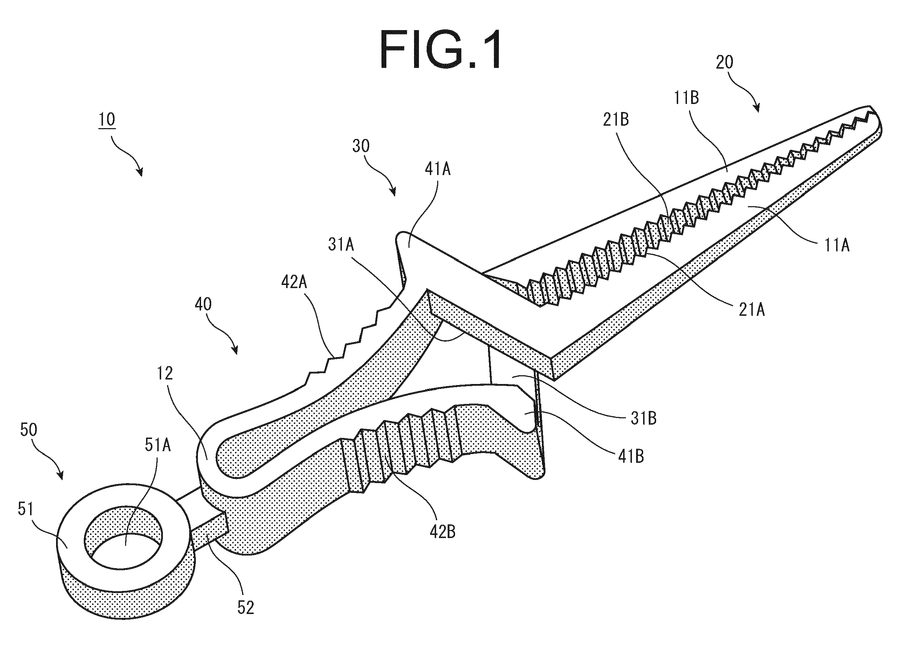

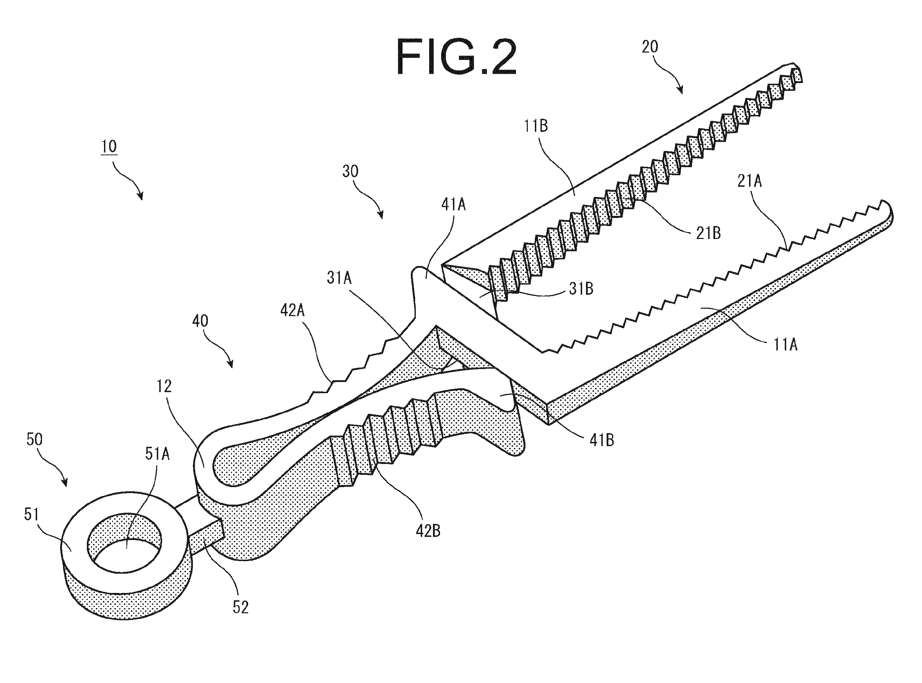

FIG.s 1 and 2 are the schematic views of the surgical clip according to one embodiment of the invention. FIG. 1 is a condition of closing the surgical clip. FIG. 2 opens the same. As shown, the surgical clip 10 of the invention is composed of a pair of feet 11A, 11B curving and connecting at a curve 12, and a balancer 50.

The feet 11A, 11B are formed with a clipping portion 20 for holding an object, a crossed portion 30 giving clipping force to a clipping portion 20 owing to elasticity of the members by their crossing, and a gripper 40 releasing the clipping portion 20 by an operator gripping it with his fingers on its outside.

Herein, the surgical clip is formed in combination of plural pieces of plastic materials including a silicone resin.

The clipping portion 20 is structured with the front ends of the feet 11A, 11B. The feet 11A, 11B have respectively objective clipping faces 21A, 21B. These clipping faces 21A, 21B are preferably formed with, e.g., concaves and convexes or fine notches to be non-slips.

A crossed portion 30 gives clipping force to the clipping portion 20 owing to elasticity of the members by crossing the feet 11A, 11B. At the portion of crossing both feet 11A, 11B, concaves 31A, 31B are formed respectively.

A gripper 40 is formed almost in loop shape with the feet 11A, 11B curving in a U-shape at a curve 12 and crossing at the crossed portion 30. Herein, the feet 11A, 11B of the gripper 40 have expansion parts 41A, 41B widening toward the outside as going to the crossed portion 30. Desirably, the gripper 40 has partly non-slips 42A, 42B as concave and convex on its surface.

The balancer 50 is a part serving as a stopper against slipping when the operator's fingers hold the gripper 40, and is formed outside of the curve 12. It is here composed of a ring 51 and a connection 52. The ring 51 is a ring-shaped member formed with a hole 51A, and connected to the curve 12 via the connection 52. The balancer 50 may be cubic, polygonal or other various shapes other than the ring shown here.

Preferably, the surgical clip 10 of the above structure is injection-molded integrally with a resin. At this time, the surgical clip 10 is molded as in FIG.s 3 and 4, not crossing the crossed portion 30. After having molded, the feet 11A, 11B are crossed respectively and an engagement is formed at the concaves 31A, 31B, and thus, the surgical clip 10 is accomplished.

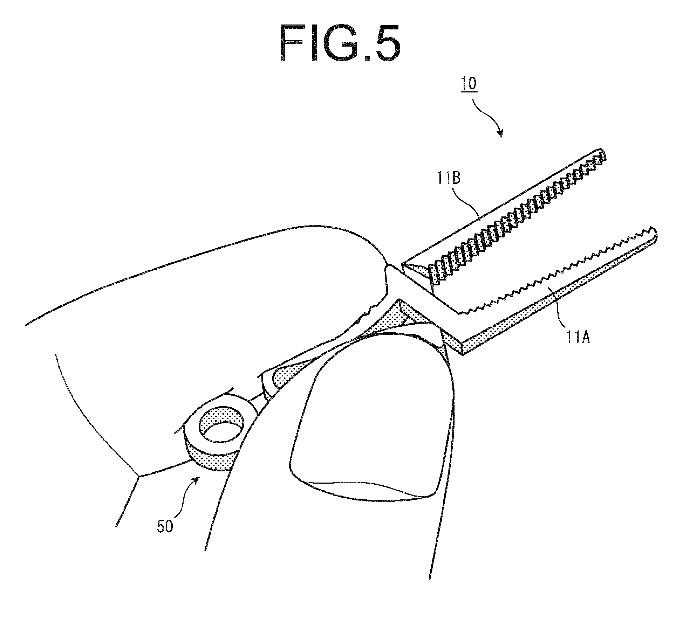

For using the above mentioned surgical clip 10, by gripping the gripper 40 on the outside in the same manner as conventionally to effect force, the feet 11A, 11B of the clipping portion 20 are released. Only, since the surgical clip 10 of the invention is furnished with the balancer 50, this can be easily handled by the operator's fingers, though not using the forceps each time. FIG. 5 shows the surgical clip 10 gripped by the operator's fingers. As shown, since the balancer 50 is placed backward the gripper 40, the finger catches easily to avoid slipping. In particular, the hole 51 is slanted reversely with respect to inclinations of the expansion parts 41A, 41B of the gripper 40, so that slipping is prevented effectively.

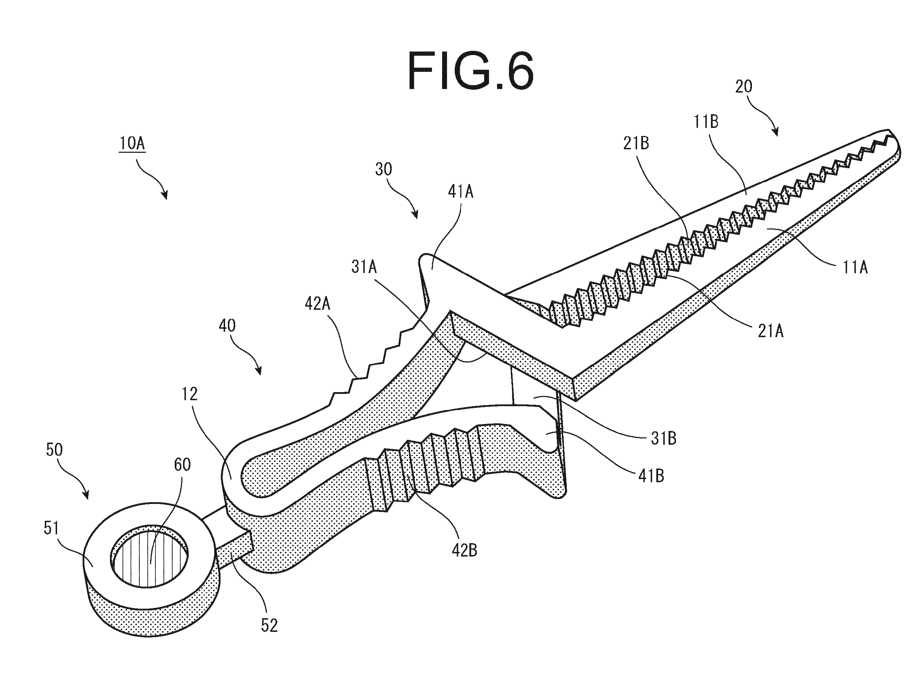

Next reference will be made to another embodiment of the invention. In the following, the surgical clip has a weight as the balancer. FIG. 6 is the perspective view of closing the surgical clip according to the other embodiment of the invention. By the way, the same part as in the embodiment shown in FIG. 1 will be given the same numerals to omit detailed explanation.

As shown in FIG. 6, the surgical clip 10A of the present embodiment is added to the balancer 50A with a ball 60 as a weight. Preferably, the ball 60 is fixedly fitted in a hole 51A of the ring 51, and the ball 60 is made of a stainless steel.

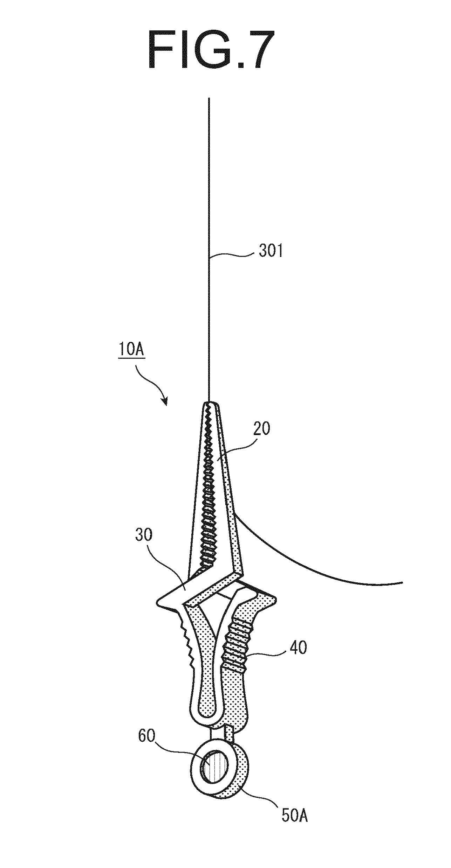

Since the conventional surgical clip is light in weight and small, it is assumed to easily split or fly when attaching or detaching. Therefore, in this embodiment, the ball 60 is employed as the weight to effect it to be moderately heavy for more easily handling the surgical clip 10A. Further, as shown in FIG. 7, when using the surgical clip 10A for clipping the suture thread 301 at its end portion, the suture thread 301 is held by suspending in straight to check a pulling-danger made by the operator. When using it for purpose of clipping the suture thread, it is enough to attach such as a cover at the front ends for slip-prevention.

The above embodiment illustrates the clipping portion straightly at the front end, and the clipping faces of the invention may employ a clipping portion having the feet bent or curved.

As mentioned above, depending on the present invention, it is possible to realize the surgical clip of the finger easily catching by means of the simple structure than the foregoing surgical clip, although not using the forceps, attaching and detaching are easy to avoid slipping.

The above explanation has been made to the embodiments of the invention, but the invention is not limited thereto, and so far as not deviating from the subject matter of the invention, various kinds of embodiments are, of course, available.

### INDUSTRIAL APPLICABILITY

The present invention relates to the surgical clip to be used for chiefly clipping a blood vessel to control blood flow, in particular, to such a surgical clip easily opening and closing without the forceps.

### EXPLANATION OF THE REFERENCE NUMERALS AND SIGNS

10, 10A: Surgical clip
11A, 11B: Feet
12: Curve
20: Clipping portion
21A, 21B: Clipping faces
30: Crossed portion
31A, 31B: Concaves
40: Gripper
41A, 41B: Expansion parts
42A, 42B: Non-slips
50, 50A: Balancer
51: Ring
51A: Hole
52: Connection
60: Ball

## Claims

1. A surgical clip, comprising a clipping portion having a pair of feet for holding an object therebetween and a crossed portion of the feet crossing each other, wherein the crossing feet are connected in loop-shape and a bridged portion connected in loop-shape is a gripper.

2. A surgical clip, comprising a clipping portion having a pair of feet for holding an object therebetween and a crossed portion of the feet crossing each other, wherein the crossing feet are connected in loop-shape, a bridged portion connected in loop-shape is a gripper, and
a balancer is further connected to the gripper.

3. A surgical clip as set forth in claim 2, wherein the balancer is further provided with a weight.

4. A surgical clip as set forth in claim 3, wherein the balancer is made of a ring shaped member, and the weight has a ball shape to be fitted in the ring shaped member.

5. A surgical clip as set forth in any of claims 1 to 4, wherein the surgical clip is formed in combination of plural pieces of plastic materials including a silicone resin.
